# EUROPEAN PATENT SPECIFICATION

(11) **EP 1 437 405 B1**
(45) Date of publication and mention of the grant of the patent: **23.12.2009**
(21) Application number: 02765543.0
(22) Date of filing: 13.09.2002
(51) Int. Cl.: C12N 15/09, C12N 15/69, C12N 1/19, C12N 1/21, C12N 5/10, C12P 21/02, C12R 1/865

(54) **GENE OVEREXPRESSION SYSTEM**
GENÜBEREXPRESSIONSSYSTEM
SYSTEME DE SUREXPRESSION GENIQUE

(30) Priority: 20.09.2001 JP 2001286637; 20.09.2001 JP 2001287159; 30.04.2002 JP 2002128323; 30.04.2002 JP 2002128286
(43) Date of publication of application: 14.07.2004
(73) Proprietor: Toyota Jidosha Kabushiki Kaisha, Toyota-shi, Aichi 471-8571 (JP)
(72) Inventor: SAITOH, Satoshi, c/o Toyota Jidosha K.K., Toyota-shi, Aichi 471-8571 (JP); SAOTOME, Osamu, c/o Toyota Jidosha K.K., Toyota-shi, Aichi 471-8571 (JP); YASUTANI, Noriko, c/o Toyota Jidosha K.K., Toyota-shi, Aichi 471-8571 (JP); MATSUO, Yasuo, c/o Toyota Jidosha Kabushiki Kaisha, Toyota-shi, Aichi 471-8571 (JP); ISHIDA, Nobuhiro, c/o K.K. TOYOTA CHUO Kenkyusho, Aichi-gun, Aichi 480-1192 (JP); HIRAI, Masana, c/o K.K. Toyota Chuo Kenkyusho, Aichi-gun, Aichi 480-1192 (JP); KITAMOTO, Katsuhiko, Ushiku-shi, Ibaraki 300-1232 (JP)
(74) Representative: TBK-Patent
(86) International application number: PCT/JP2002/009452
(87) International publication number: WO 2003/027280

(56) References cited:
- WO-A1-00/70065
- FR-A- 2 793 805
- US-A- 5 631 143
- DOHMEN R J ET AL: "CLONING OF THE SCHWANNIOMYCES-OCCIDENTALIS GLUCOAMYLASE GENE GAM1 AND ITS EXPRESSION IN SACCHAROMYCES-CEREVISIAE" GENE (AMSTERDAM), vol. 95, no. 1, 1990, pages 111-122, XP002297355 ISSN: 0378-1119
- EBERHARDT INES ET AL: "Autoregulation of yeast pyruvate decarboxylase gene expression requires the enzyme but not its catalytic activity" EUROPEAN JOURNAL OF BIOCHEMISTRY, vol. 262, no. 1, May 1999 (1999-05), pages 191-201, XP002297356 ISSN: 0014-2956
- KELLERMANN E ET AL: "Analysis of the primary structure and promoter function of a pyruvate decarboxylase gene (PDC1) from saccharomyces cerevisiae" NUCLEIC ACIDS RESEARCH, OXFORD UNIVERSITY PRESS, SURREY, GB, vol. 14, no. 22, 1986, pages 8963-8977, XP002961301 ISSN: 0305-1048
- DESTRUELLE M ET AL: "Regulation of the expression of the Kluyveromyces lactis PDC1 gene: carbon source-responsive elements and autoregulation" YEAST, CHICHESTER, SUSSEX, GB, vol. 15, no. 5, 1999, pages 361-370, XP002961304 ISSN: 0749-503X
- GELLISSEN GERD ET AL: "Application of yeasts in gene expression studies: A comparison of Saccharomyces cerevisiae, Hansenula polymorpha and Kluyveromyces lactis: A review" GENE (AMSTERDAM), vol. 190, no. 1, 1997, pages 87-97, XP002297357 ISSN: 0378-1119
- MENDOZA-VEGA O ET AL: "Industrial production of heterologous proteins by fed-batch cultures of the yeast Saccharomyces cerevisiae" FEMS MICROBIOLOGY REVIEWS 1994 NETHERLANDS, vol. 15, no. 4, 1994, pages 369-410, XP002297358 ISSN: 0168-6445
- CHO Y-W ET AL: "Characterization and regulation of Schizosaccharomyces pombe gene encoding thioredoxin" BIOCHIMICA ET BIOPHYSICA ACTA . GENE STRUCTURE AND EXPRESSION, ELSEVIER, AMSTERDAM, NL, vol. 1518, no. 1-2, 19 March 2001 (2001-03-19), pages 194-199, XP004275875 ISSN: 0167-4781
- GAN Z R: "Yeast thioredoxin genes." THE JOURNAL OF BIOLOGICAL CHEMISTRY. 25 JAN 1991, vol. 266, no. 3, 25 January 1991 (1991-01-25), pages 1692-1696, XP002305703 ISSN: 0021-9258
- GREY M ET AL: "Overexpression of ADH1 confers hyper-resistance to formaldehyde in Saccharomyces cerevisiae" CURRENT GENETICS, NEW YORK, NY, US, vol. 29, 1996, pages 437-440, XP002125609 ISSN: 0172-8083
- DRACULIC T ET AL: "A single glutaredoxin or thioredoxin gene is essential for viability in the yeast Saccharomyces cerevisiae." MOLECULAR MICROBIOLOGY. JUN 2000, vol. 36, no. 5, June 2000 (2000-06), pages 1167-1174, XP002305704 ISSN: 0950-382X
- KELLERMANN E. ET AL.: 'Analysis of the primary structure and promoter function of a pyruvate decarboxylase gene (PDC1) from saccharomyces cerevisiae' NUCLEIC ACIDS RES. vol. 14, no. 22, 1986, pages 8963 - 8977, XP002961301
- BUTLER G. ET AL.: 'Identification of an upstream activation site in the pyruvate decarboxylase structural gene (PDC1) of saccharomyces cerevisiae' CURR. GENET. vol. 14, no. 5, 1988, pages 405 - 412, XP002961302
- EBERHARDT I. ET AL.: 'Autoregulation of yeast pyruvate decarboxylase gene expression requires the enzyme but not its catalytic activity' EUR. J. BIOCHEM. vol. 262, no. 1, 1999, pages 191 - 201, XP002961303
- DESTRUELLE M. ET AL.: 'Regulation of the expression of the Kluyveromyces lactis PDC1 gene: carbon source-responsive elements and autoregulation' YEAST vol. 15, no. 5, 1999, pages 361 - 370, XP002961304
- SEEBOTH P.G. ET AL.: 'PDC1(0) mutants of saccharomyces cerevisiae give evidence for an additional structural PDC gene: cloning of PDC5, a gene homologous to PDC1' J. BACTERIOL. vol. 172, no. 2, 1990, pages 678 - 685, XP002961305
- LIESEN T. ET AL.: 'ERA, a novel cis-acting element required for autoregulation and ethanol repression of PDC1 transcription in saccharomyces cerevisiae' MOL. MICROBIOL. vol. 21, no. 3, 1996, pages 621 - 632, XP002961306
- HOHMANN S.: 'PDC6, a weakly expressed pyruvate decarboxylase gene from yeast, is activated when fused spontaneously under the control of the PDC1 promoter' CURR. GENET. vol. 20, no. 5, 1991, pages 373 - 378, XP002961307
- LIM C.J. ET AL.: 'Growth-phase regulation of the escherichia coli thioredoxin gene' BIOCHIM. BIOPHYS. ACTA vol. 1491, no. 1-3, 2000, pages 1 - 6, XP004275608
- PASTERNAK C. ET AL.: 'Expression of the thioredoxin gene (trxA) in rhodobacter sphaeroides Y is regulated by oxygen' MOL. GEN. GENET. vol. 250, no. 2, 1996, pages 189 - 196, XP002961309

### SEQUENCE LISTING

<110> TOYOTA JIDOSHA KABUSHIKI KAISHA, KABUSHIKI KAISHA TOYOTA CHUO KENKYUSHO
<120> Gene Expression System
<130> PH-1638PCT
<150> JP 2001-286637 <151> 2001-09-20
<150> JP 2001-287159 <151> 2001-09-20
<150> JP 2002-128323 <151> 2002-04-30
<150> JP 2002-128286 <151> 2002-04-30
<160> 8
<170> PatentIn Ver. 2.0
<210> 1
   <211> 971
<212> DNA
   <213> Saccharomyces cerevisiae
<400> 1
   aagggtagcc tccccataac ataaactcaa taaaatatat agtcttcaac ttgaaaaagg 60
   aacaagctca tgcaaagagg tggtacccgc acgccgaaat gcatgcaagt aacctattca 120
   aagtaatatc tcatacatgt ttcatgaggg taacaacatg cgactgggtg agcatatgct 180
   ccgctgatgt gatgtgcaag ataaacaagc aagacggaaa ctaacttctt cttcatgtaa 240
   taaacacacc ccgcgtttat ttacctatct ttaaacttca acaccttata tcataactaa 300
   tatttcttga gataagcaca ctgcacccat accttcctta aaagcgtagc ttccagtttt 360
   tggtggttcc ggcttccttc ccgattccgc ccgctaaacg catatttttg ttgcctggtg 420
   gcatttgcaa aatgcataac ctatgcattt aaaagattat gtatgctctt ctgacttttc 480
   gtgtgatgaa gctcgtggaa aaaatgaata atttatgaat ttgagaacaa ttctgtgttg 540
   ttacggtatt ttactatgga ataattaatc aattgaggat tttatgcaaa tatcgtttga 600
   atatttttcc gaccctttga gtacttttct tcataattgc ataatattgt ccgctgcccg 660
   tttttctgtt agacggtgtc ttgatctact tgctatcgtt caacaccacc ttattttcta 720
   actatttttt ttttagctca tttgaatcag cttatggtga tggcacattt ttgcataaac 780
   ctagctgtcc tcgttgaaca taggaaaaaa aaatatataa acaaggctct ttcactctcc 840
   ttgcaatcag atttgggttt gttcccttta ttttcatatt tcttgtcata ttcctttctc 900
   aattattatt ttctactcat aaccacacgc aaaataacac agtcaaatca atcaaagatc 960
   ccccaattct c 971
<210> 2
   <211> 31
   <212> DNA
   <213> Artificial Sequence
<220>
   <223> Description of Artificial Sequence: primer
<400> 2
   atatatggat ccgcgtttat ttacctatct c 31
<210> 3
   <211> 31
   <212> DNA
   <213> Artificial Sequence
<220>
   <223> Description of Artificial Sequence: primer
<400> 3
   atatatgaat tctttgattg atttgactgt g 31
<210> 4
   <211> 34
   <212> DNA
   <213> Artificial Sequence
<220>
   <223> Description of Artificial Sequence: primer
<400> 4
   atatatctcg aggccagcta acttcttggt cgac 34
<210> 5
   <211> 31
   <212> DNA
   <213> Artificial Sequence
<220>
   <223> Description of Artificial Sequence: primer
<400> 5
   atatatgaat tctttgattg atttgactgt g 31
<210> 6
   <211> 32
   <212> DNA
   <213> Artificial Sequence
<220>
   <223> Description of Artificial Sequence: primer
<400> 6
   aaatttgtcg acaagggtag cctccccata ac 32
<210> 7
   <211> 31
   <212> DNA
   <213> Artificial Sequence
<220>
   <223> Description of Artificial Sequence: primer
<400> 7
   atatatgtcg acgagaattg ggggatcttt g 31
<210> 8
   <211> 43
   <212> DNA
   <213> Artificial Sequence
<220>
   <223> Description of Artificial Sequence: primer
<400> 8
   tttaaagtcg actttgattg atttgactgt gttattttgc gtg 43

## Description

### FIELD OF THE INVENTION

The present invention relates to a method of producing lactic acid by expressing a lactate dehydrogenase gene in Saccharomyces cerevisiae.

### BACKGROUND ART

When substances are produced by a host organism, or the function of a host organism is altered or analyzed, a genetic engineering technique is employed. This involves introducing a homologous or heterologous gene into the host organism for expression. However, this genetic engineering technique is not sufficient in terms of stability and high expression, and thus there have been expectations that it would be improved.

For example, when a host organism is yeast *Saccharomyces cerevisiae,* a YEP type vector utilizing a 2-µm DNA is often used. The YEP vector enables introduction of a large number of copies of a gene. However, the YEP vector cannot be said to be sufficient in terms of stability because an enzyme activity of the product from the introduced gene may decrease due to, for example, the loss of the vector during cell division. To improve the enzyme activity, a vector is designed to contain a drug resistance marker and the drug is added to a medium, or designed to contain an auxotrophic marker when the auxotrophic marker has already been provided in a host strain, and selection pressure can be applied by further utilizing a highly purified minimum medium (YNB, Difco) as a medium. However, all such cases are disadvantageous in that the medium cost is expensive.

In the meantime, as a vector that can integrate a gene into a chromosome, a YIP type vector utilizing homologous recombination is known. This YIP vector enables a transgene to be present stably on the genome depending on the design of the vector, but in general it is unable to achieve high expression of the transgene.

From the reasons described above, a method of highly expressing a gene by introducing or inserting the gene into a host organism stably and at low cost has been desired in the art.

Furthermore, for example when a target gene is expressed in a yeast cell *Saccharomyces cerevisae,* it is required to ligate a promoter that can be expressed within yeast upstream of the gene. As a currently reported promoter for *Saccharomyces cerevisae,* the promoter of the alcohol dehydrogenase 1 (ADH1) gene and the promoter of the 3-phosphoglycerate kinase (PGK) gene are known to show strong expression levels. Moreover, gene transfer by homologous recombination leads to high stability of the gene. Hence, if a target gene can be expressed under a strong promoter, it would be efficient in producing a substance, and altering and analyzing the function.

However, when a gene is inserted into yeast by homologous recombination, the above YIP vector is utilized. In this case, the number of copies that can be expected is only 1 or 2. Thus, development of a promoter that enables high expression even with a single copy has been desired.

DOHMEN R J ET AL: "CLONING OF THE SCHWANNIOMYCES-OCCIDENTALIS GLUCOAMYLASE GENE GAM1 AND ITS EXPRESSION IN SACCHAROMYCES-CEREVISIAE" GENE (AMSTERDAM), vol. 95, no. 1, 1990, discloses cloning of glucoamylase (GAM)-encoding gene (GAM1) from *Schwanniomyces* occidentalis and use of PDC1 promoter to express GAM1 gene in *Saccharomyces cerevisiae*. The GAM1 gene was introduced by using 2µm plasmid vector pYc1 but it was not integrated into the host chromosome.

KELLERMANN E ET AL: "Analysis of the primary structure and promoter function of a pyruvate decarboxylase gene (PDC1) from saccharomyces cerevisiae" NUCLEIC ACIDS RESEARCH, OXFORD UNIVERSITY PRESS, SURREY, GB, vol. 14, no. 22, 1986 reports the function of a promoter region of PDC1 (pyruvate decarboxylase 1) gene. In this study, the ß-lactamase (bla) gene was introduced into yeast such that the bla gene was inserted downstream of the PDC1 gene or the gene wass fused with the PDC1 gene.

US-A-5 631 143 discloses a promoter of PDC1 (pyruvate decarboxylase 1) gene from Kluyveromyces lactis (D4, Abstract). Further, use of the promoter for expression of a heterologous gene is described.

### SUMMARY OF THE INVENTION

An object of the present invention is to provide a method of producing lactic acid as defined in claim 1.

As a result of thorough studies to achieve the above objects, we have found that a lactate dehydrogenase gene as a target gene can be stably and highly expressed by ligating the target gene to be expressed to a pyruvate decarboxylase 1 promoter in yeast *Saccharomyces cerevisae,* and then integrating the gene into the genome of a host.

Furthermore, we have focused on the event that an extremely large quantity of ethanol is produced in *Saccharomyces cerevisae.* We have expected that the pyruvate decarboxylase 1 gene is highly expressed in the ethanol fermentation pathway, and isolated the promoter region of the above pyruvate decarboxylase 1 (PDC1) gene. Furthermore, we have ligated the promoter region of PDC1 gene to a lactate dehydrogenase gene as a target gene, and inserted the gene into *Saccharomyces cerevisae,* thereby obtaining a finding that the target gene is highly expressed under the promoter. Based on the above findings, we have completed the present invention.

In the present invention, the above promoter of a gene wherein an autoregulation mechanism is present is the promoter of the pyruvate decarboxylase 1 gene. The promoter of a gene that is not essential for growth includes, for example, the promoter of a gene encoding thioredoxin.

The host organism is *Saccharomyces* cerevisiae. These host organisms also mean any of individual organisms (excluding humans), tissues and cells.

Furthermore, in the present invention, the above promoter of the pyruvate decarboxylase 1 gene is a promoter comprising any one of the following DNAs (a) to (c):
(a) a DNA, comprising the nucleotide sequence represented by SEQ ID NO: 1;
(b) a DNA, comprising a nucleotide sequence derived from the nucleotide sequence represented by SEQ ID NO: 1 by deletion, substitution or addition of 1 to 40 nucleotides, and having a promoter activity of the pyravate decarboxylase gene from 8. cerevisiae ; and
(c) a DNA, capable of hybridizing under stringent conditions to a DNA comprising a sequence complementary to the whole or a part of the nucleotide sequence represented by SEQ ID NO: 1, and having a promoter activity of the pyravate decarboxylase gene from 8 cerevisiae.

The present invention is explained in detail as follows. This application claims a priority from Japanese Patent Application No. 2001-286637 filed September 20, 2001 and Japanese Patent Application No. 2002-128323 filed April 30, 2002 which claims a priority from the aforementioned application, and Japanese Patent Application No. 2001-287159 filed September 20, 2001, and Japanese Patent Application No. 2002-128286 filed April 30, 2002, which claims a priority from the aforementioned application.

In the living world, some have genes wherein an autoregulation mechanism is present and genes that are not essential for growth or fermentation. We have focused on this point, and selected promoters of such genes for methods of gene transfer and gene expression. Hence, the gene expression method of the present invention comprises inserting a target gene into a genome under the control of the promoter of a gene wherein an autoregulation mechanism is present or the promoter of a gene that is not essential for growth or fermentation in a host organism. The method of the present invention is as summarized as follows.

### 1. Selection of promoter

First, the promoter of a gene wherein an autoregulation mechanism is present, or the promoter of a gene that is not essential for growth or fermentation of a host organism is selected. As a target host organism, all organisms that are expected to produce substances, and to alter their function or to analyze their function, can be used as host organisms. Examples of a host organism include bacteria, yeast, insects, animals and plants.

### (1) Gene promoter wherein an autoregulation mechanism is present

To select the promoter of a gene wherein an autoregulation mechanism is present, a gene wherein the autoregulation mechanism is present is first specified. "Autoregulation mechanism" means a mechanism wherein a plurality of genes having the same function is present in the same organism, of which at least one gene is normally expressed and the remainders are suppressed, and the remaining genes are expressed to continue the function only when the generally expressed gene becomes unable to function because of disruption or the like. For example, the pyruvate decarboxylase (PDC) gene of yeast belonging to the genus *Saccharomyces* is a gene encoding an enzyme that converts by decarboxylating pyruvic acid into acetaldehyde in the process of ethanol synthesis, and plays an important role in the fermentation process. PDC includes PDC1, PDC5 and PDC6, but normally PDC1 is activated, and PDC5 and PDC6 are suppressed by the action of PDC1. However, when gene disruption or a mutation caused by a drug occurs to PDC 1 so as to inactivate the function thereof, the PDC5 gene is activated, whereby the ethanol-producing function of yeast is not lost (Eberhardt, 1. et al., Eur. J. Biochem. 1999, 262(1): 191-201 ; Muller, EH. et al., FEBS Lett. 1999, 449 (2-3): 245-250). Actually, Schaaff et al., have deleted the PDC1 promoter, and then confirmed the pyruvate decarboxylase activity of yeast (Schaaff, I. et al., Curr. Genet. 1989, 15:75-81). Specifically, the phenotype is almost equivalent to that of the parent strain. On the other hand, the PDC 1 promoter has been isolated in the genus *Kluyveromyces* classified as yeast (International publication WO 94/01569). However, the autoregulation mechanism has not been reported in the genus *Kluyveromyces.*

A gene wherein the autoregulation mechanism is present can be specified by confirming, when a gene is disrupted in a strain, if a protein encoded by the gene is still expressed. The promoter of the thus specified gene wherein the autoregulation mechanism is present is selected. In the present invention the promoter of PDC1 (hereinafter referred to as the PDC 1 promoter) is selected.

### 2. Preparation of target gene and promoter

The above-selected promoter and a target gene to be inserted into a host organism are prepared. In the present invention, "target gene" means gene encoding lactate dehydrogenase, which produces lactic acid from pyruvic acid.

For the preparation of a target gene and the above promoter, any technique known in the art can be employed. For example, when the above target gene and the above promoter are isolated from a source, the above target gene and the above promoter can be prepared by a method for synthesizing cDNA from RNA that has been prepared by a guanidine isothiocyanate method. In addition, the above target gene and the above promoter can also be prepared by amplification by PCR using a genomic DNA as a template. The thus obtained DNAs of the above target gene and the above promoter can be used directly depending on the purpose, or can be used after digestion with a restriction enzyme or after addition of a linker, if desired.

In the present invention, a DNA containing a sequence derived from the nucleotide sequence of the promoter by deletion, substitution or addition of 1 to 40 nucleotides, and having promoter activity as specified above can also be utilized as a promoter. Promoter activity means to have the ability and function of causing a target gene to produce gene products in a host or outside the host when the target gene is inserted into the host by operably linking the target gene downstream to the promoter. In such a DNA, the promoter activity is maintained at a level that enables almost the same application thereof under the same conditions as those for a promoter comprising a full-length nucleotide sequence without any mutations (deletion, substitution or addition) to function. For example, such a DNA maintains promoter activity that is approximately 0.01 to 100 times, preferably approximately 0.5 to 20 times, and more preferably approximately 0.5 to 2 times greater than that of a full-length sequence.

Such a DNA can be produced as described in literature such as Molecular Cloning (Sambrook, et al., ed., (1989) Cold Spring Harbor Lab. Press, New York).

For example, by the technology in 1 to 40 nucleotides is(are) deleted, substituted or added based on and from the nucleotide sequence of the promoter of a gene wherein the autoregulation mechanism is present, or the nucleotide sequence of the promoter of a gene that is not essential for growth or fermentation in a host organism, for example by the site-directed mutagenesis method, a variant having a different sequence while maintaining promoter activity can be prepared. For example, for site-directed mutagenesis whereby 1 to 40 nucleotides are substituted, a variant can be obtained according to the technology described in literature such as Proc. Natl. Acad. Sci. USA 81 (1984) 5662-5666; International Publication No. WO85/00817; Nature 316 (1985) 601-605; Gene 34 (1985) 315-323; Nucleic Acids Res. 13 (1985) 4431-4442; Proc. Natl. Acad. Sci. USA 79(1982) 6409-6413; or Science 224 (1984) 1431-1433, and then the variant can be utilized. In addition, these variants can be prepared using a commercially available kit (Mutan-G and Mutan-K (TAKARA BIO)). Furthermore, error-prone polymerase chain reaction (error-prone PCR) is also known as a method for preparing variants, and by selecting a condition wherein the degree of accuracy for replication is low, a mutation of 1 to several nucleotides can be introduced (Cadwell, R.C. and Joyce, G.F. PCR Methods and Applications 2(1992) 28-33; Malboeuf, C. M. et al. Biotechniques 30(2001) 1074-8; Moore, G.L. and Maranas C.D. J. Theor. Biol. 7; 205 (2000) 483-503).

Furthermore, hybridization under stringent conditions using, as a probe (100 to 900 nucleotides), a DNA comprising a sequence complementary to the whole or a part of the nucleotide sequence of the promoter of a gene wherein the autoregulation mechanism is present, or the promoter of a gene that is not essential for growth or fermentation in a host organism enables to newly obtain and utilize a DNA that has a function (that is, promoter activity) similar to that of a DNA comprising the nucleotide sequence of the promoter of a gene wherein the autoregulation mechanism is present, or the promoter of a gene that is not essential for growth or fermentation in a host organism, and comprises another nucleotide sequence. Here, stringent conditions mean conditions wherein, for example, the sodium concentration is between 10 and 300 mM, and preferably between 20 and 100 mM, and the temperature is between 25 and 70°C, and preferably between 42 and 55°C.

Whether or not a variant obtained as described above and a DNA obtained by hybridization have activity as promoters can be confirmed by the following procedures. Specifically, the promoter activity of the DNA obtained as described above can be confirmed by preparing a vector wherein, preferably, various reporter genes, for example, the luciferase (LUC) gene, the chloramphenicol acetyltransferase (CAT) gene and the β-galactosidase (GAL) gene are ligated to the downstream region of the promoter, inserting the genes into a host genome using the vector, and then measuring the expression of the reporter genes.

### 3. Insertion of target gene and promoter

Subsequently, the above gene wherein the autoregulation mechanism is present is disrupted, and then a target gene is inserted under the control of the promoter of the gene, or alternatively, the gene is substituted with a target gene.

For example, the target gene isolated as described above is operably linked to the promoter selected as described above, and then inserted into the genome of a host organism. "Operably linked to" means that a target gene is linked to the above promoter so that the target gene is expressed under the control of the above promoter in a host organism to which the target gene is inserted. A target gene and the above promoter can be inserted using any technique known in the art. For example, a target gene and the above promoter can be inserted into the genome of a host organism using a recombinant vector. A recombinant vector can be obtained by ligating (inserting) a target gene and the above promoter to an appropriate vector. Examples of a vector for the insertion of a target gene are not specifically limited, as long as they can be integrated into the genome in a host organism, and include a plasmid DNA, a bacteriophage DNA, a retrotransposon DNA and a yeast artificial chromosome DNA (YAC).

Examples of a plasmid DNA include YIp-type *Escherichia coli*-yeast shuttle vectors such as pRS403, pRS404, pRS405, pRS406, pAUR101 or pAUR135; plasmids derived from *Escherichia coli* (ColE plasmid such as pBR322, pBR325, pUC18, pUC19, pUC118, pUC119, pTV118N, pTV119N, pBluescript, pHSG298, pHSG396 or pTrc99A; a p15A plasmid such as pACYC177 or pACYC184; or a pSC101 plasmid such as pMW118, pMW119, pMW218 or pMW219); and plasmids derived from *Bacillus* (e.g., pUB110 or pTP5). Examples of a phage DNA include λ phage (Charon4A, Charon21A, EMBL3, EMBL4, ?gt10, ?gt11 or ?ZAP), fX174, M13mp18 and M13mp19. An example of retrotransposon is Ty factor. An example of a vector for YAC is pYACC2.

To insert the above target gene and the above promoter into a vector, for example, a method that is employed herein involves, first, cleaving a purified DNA with an appropriate restriction enzyme, and then inserting the product at the restriction site or the multi-cloning site of an appropriate vector DNA so as to ligate the product to the vector.

The above target gene should be incorporated into a vector so that the function of the gene is exerted under the control of the above-selected promoter. Hence, in addition to the above-selected promoter, the above target gene and a terminator, cis element such as an enhancer, splicing signal, polyA addition signal, ribosome binding sequence (SD sequence) and the like can be ligated to a recombinant vector, if desired. Furthermore, a selection marker indicating that the vector is retained within the cell may also be ligated. In addition, examples of a selection marker include the dihydrofolate reductase gene, the ampicillin resistance gene and the neomycin resistance gene. In addition, an example of a marker gene is the gene for tryptophan synthesis (TRP1 gene), but is not limited thereto. Other marker genes, for example, the URA3 gene, the ADE2 gene and the HIS3 gene having auxotrophic ability, or the G418 resistance gene having drug resistance ability can also be utilized.

An example of a terminator sequence is the terminator gene of the glyceraldehyde-3-phosphate dehydrogenase gene (GAPDH), but is not limited thereto in the present invention. Any terminator sequence may be used, as long as it is a terminator sequence that can be used within a host organism.

As described above, a recombinant vector can be prepared so as to be applicable for the expression of the above target gene in a host organism. By transforming a host organism using the recombinant vector, the above target gene can be expressed under the control of the above-selected promoter in the host organism. *Saccharomyces cerevisiae*,

is used. A method for introducing a recombinant vector into this yeast is not specifically limited, as long as it is a method for introducing a DNA into yeast. Examples of such a method include an electroporation method, a spheroplast method and a lithium acetate method.

A host organism wherein a recombinant vector is introduced as described above is subjected to selection for strains (clones) having a target gene introduced under the control of the above-selected promoter. Specifically, transformant are selected using the above selection marker as an indicator.

Whether or not a target gene is incorporated under the control of the above promoter can be confirmed by the PCR (polymerase chain reaction) or the Southern hybridization. For example, a DNA is prepared from a transformant, introduced DNA-specific primers are designed, and then PCR is performed using the primers and prepared DNA. Subsequently, the amplification product is subjected to agarose gel electrophoresis, polyacrylamide gel electrophoresis or capillary electrophoresis, stained with ethidium bromide, SYBR Green solution or the like and then detected as a single band, so that the introduced DNA can be confirmed. Furthermore, PCR is performed using primers previously labeled with fluorescent dye or the like, so that an amplification product can be detected. Furthermore, a method that can be also employed herein involves binding an amplification product to a solid phase such as a microplate, and then confirming the amplification product by fluorescence reaction, enzyme reaction or the like.

As described above, under the control of the promoter of a gene wherein the autoregulation mechanism is present, or the promoter of a gene that is not essential for growth or fermentation of a host organism, a target gene is inserted into a genome (genome integration), so that the target gene is expressed in the host organism. Since the PDC1 promoter is a very strong promoter, when the PDC 1 promoter is selected, a target gene is highly expressed even when inserted in the form of a single copy into the genome. Furthermore, since the endogenous gene of which the promoter is selected is not essential for growth and fermentation, even when it is disrupted or substituted with a target gene, the host organism can continue the growth and the fermentation so as to be able to express the target gene for a long time period.

### 4. Pyruvate decarboxylase 1 gene (PDC1) promoter

The promoter of the present invention is a promoter (hereinafter, referred to as the PDC1 promoter) of pyruvate decarboxylase 1 gene isolated from *Saccharomyces cerevisiae.* Pyruvate decarboxylase is an enzyme involved in the ethanol fermentation pathway of yeast. In general, only PDC1 functions among PDC1, PDC5 and PDC6 genes (see "1. Selection of promoter" section). We focused on the fact that although pyruvate decarboxylase is produced by the expression of only PDC1 because of the autoregulation mechanism, ethanol is produced in a large quantity, and then specified the promoter region of PDC1.

The PDC1 promoter was determined and isolated as described below. First by the use of the public genome database of *Saccharomyces cerevisiae* (*Saccharomyces* Genome Database), a vector for homologous recombination was constructed so that a target gene could be inserted downstream of PDC1 promoter. This vector was introduced, strains with high expression amounts of the target gene were selected, PDC1 promoter fragments were obtained by PCR, and then the nucleotide sequence of a putative region corresponding to PDC1 promoter was determined by a sequencer (ABI 310 Genetic Analyzer).

The PDC1 promoter contains a DNA comprising the nucleotide sequence represented by SEQ ID NO: 1. After isolation of the PDC 1 promoter, the DNA can be obtained by chemical synthesis according to a technique for nucleic acid synthesis.

Moreover, the PDC1 promoter of the present invention also includes a DNA comprising a nucleotide sequence isolated from the nucleotide sequence represented by SEQ ID NO: 1 by deletion, substitution or addition of 1 to 40 nucleotides, and having promoter activity. Promoter activity means to have the ability and function of producing the gene product of a target gene within a host or outside a host when the target gene is inserted into a host by operably linked the target gene downstream to the promoter. In such a DNA, the promoter activity is maintained at a level that enables almost the same applications thereof under the same conditions as those for a promoter comprising the nucleotide sequence represented by SEQ ID NO: 1 to function. For example, such a DNA maintains promoter activity that is approximately 0.01 to 100 times, preferably approximately 0.5 to 20 times, and more preferably approximately 0.5 to 2 times greater than that of the DNA comprising the nucleotide sequence represented by SEQ ID NO: 1.

Such a DNA can be produced as described in literature such as Molecular Cloning (Sambrook et al., ed., (1989) Cold Spring Harbor Lab. Press, New York) by referring to the nucleotide sequence represented by SEQ ID NO: 1.

For example, by the technology in which 1 to 40 nucleotides is(are) deleted, substituted or added based on and from the above-described nucleotide sequence represented by SEQ ID NO: 1, such as the site-directed mutagenesis method as described in the above "2. Preparation of target gene and promoter" section, a variant having a different sequence can be prepared while maintaining promoter activity.

Furthermore, hybridization under stringent conditions using, as a probe (100 to 900 nucleotides), a DNA comprising a sequence complementary to the whole or a part of the nucleotide sequence represented by SEQ ID NO: 1 enables to newly obtain and utilize a DNA that has a function (that is, promoter activity) similar to that of the DNA comprising the nucleotide sequence represented by SEQ ID NO: 1, but which comprises another nucleotide sequence. Here, stringent conditions mean conditions wherein, for example, the sodium concentration is between 10 and 300 mM, and preferably between 20 and 100 mM, and the temperature is between 25 and 70°C, and preferably between 42 and 55°C.

Whether or not a variant obtained as described above or a DNA obtained by hybridization has activity as a promoter can be confirmed by techniques as described in the above "2. Preparation of target gene and promoter" section.

The PDC1 promoter of the present invention can be used not only for expressing a target gene under the control of the promoter utilizing the autoregulation mechanism, but also as a general promoter.

### 5. Construction of recombinant vector

The promoter of the present invention can be used as a general promoter, so that it can be utilized as a promoter to achieve high expression of a target gene. The recombinant vector of the present invention is obtained by ligating (inserting) the PDC1 promoter of the present invention and a target gene into an appropriate vector.

In order to produce a substance, a nucleic acid encoding lactate dehydrogenase is used as a target gene. Examples of such a useful protein include interferons and vaccines. The nucleic acid is the nucleic acid of a gene encoding lactate dehydrogenase for the generation of lactic acid from pyruvic acid.

A vector for the insertion of the above target gene is not specifically limited, as long as it is a vector of a type to be integrated into a chromosome, which can integrate the above target gene into the genome of a host organism as described in the above "Insertion of target gene and promoter" section, or a plasmid-type vector known in the art. Examples of such a vector include a plasmid DNA, a bacteriophage DNA, a retrotransposon DNA and yeast artificial chromosome DNA (YAC).

Examples of a plasmid DNA include YCp-type *Escherichia coli*-yeast shuttle vectors such as pRS413, pRS414, pRS415, pRS416, YCp50, pAUR112 or pAUR123, YEp-type *Escherichia coli*-yeast shuttle vector such as pYES2 or YEp13, YIp-type *Escherichia coli*-yeast shuttle vector such as pRS403, pRS404, pRS405, pRS406, pAUR101 or pAUR135, plasmids derived from *Escherichia coli* (e.g., ColE plasmids such as pBR322, pBR325, pUC18, pUC19, pUC118, pUC119, pTV118N, pTV119N, pBluescript, pHSG298, pHSG396 or pTrc99A; p15A plasmids such as pACYC177 or pACYC184; or a pSC101 plasmids such as pMW118, pMW119, pMW218 or pMW219), and plasmids derived from *Bacillus subtilis* (e.g., pUB110 or pTP5). Examples of a phage DNA include λ phage (Charon4A, Charon21A, EMBL3, EMBL4, ?gt10, ?gt11 or ?ZAP), fX174, M13mp18 and M13mp19. An example of retrotransposon is a Ty factor. An example of a vector for YAC is pYACC2.

To insert the PDC1 promoter of the present invention and the above target gene into a vector, for example, a method that involves first cleaving a purified DNA with an appropriate restriction enzyme, and then inserting the product into a restriction site or a multi-cloning site of an appropriate vector DNA, so as to ligate the product to the vector may be used.

The PDC 1 promoter of the present invention should be incorporated into a vector, so that it operably expresses the above target gene to exert the function of the above target gene. "Operably express" means that the above target gene and the PDC1 promoter are ligated to each other, and then they are incorporated into a vector, so that the target gene is expressed under the control of the PDC1 promoter in a host organism into which the target gene is inserted. Hence, to the vector of the present invention, a *cis* element such as an enhancer, splicing signal, poly A addition signal, a selection marker, ribosome binding sequence (SD sequence) or the like can be ligated, if necessary, in addition to the PDC1 promoter, the above target gene and a terminator. Furthermore, examples of a selection marker include the dihydrofolate reductase gene, the ampicillin resistance gene and the neomycin resistance gene.

### 6. Transformation with recombinant vector

The transformant of the present invention can be obtained by introducing the recombinant vector of the present invention into *Saccharomyces cerevisiae* so that a target gene can be expressed under the control of the PDC1 promoter.

A method for introducing a recombinant vector into yeast is not specifically limited, as long as it is a method for introducing a DNA into yeast.

Host organisms into which a recombinant vector has been introduced as described above are subjected to selection for strains (clones) in which a target gene has been introduced under the control of the above-selected promoter. Specifically, transformant are selected using the above selection marker as an indicator. The thus obtained transformants can highly and stably express the above target gene under the control of the PDC promoter, so that the transformant can be utilized for producing a protein encoded by the target gene as described below, or for other purposes, such as the functional analysis of the target gene.

### 7. Production of gene expression product or substance produced by expression product

Next, a method for producing a gene expression product or a substance produced by the expression product is described. In the present invention, a gene expression product or a substance produced by the expression product can be obtained by culturing the transformant obtained as described above, and collecting a gene expression product or a substance produced by the expression product from the obtained culture. "Culture" means any of culture cells or cultured organisms, or disrupted cells or disrupted organisms, in addition to culture supernatant. The method of culturing the transformant of the present invention is performed according to a normal method applied for culturing a host.

As a medium for culturing transformants obtained using S. cerevisiae as a host, either a natural medium or a synthetic medium can be used, as long as the medium contains a carbon source, a nitrogen source, inorganic salts and the like that microorganisms can utilize, and enables effective culture of the transformants. As a carbon source, carbohydrate such as glucose, fructose, sucrose or starch, an organic acid such as acetic acid or propionic acid, or alcohols such as ethanol or propanol may be used. As a nitrogen source, ammonium salts of inorganic acid or organic acid such as ammonia, ammonium chloride, ammonium sulfate, ammonium acetate or ammonium phosphate, or other nitrogen-containing compounds, as well as peptone, meat extract, corn steep liquor or the like may be used. As an inorganic substance, potassium primary phosphate, potassium secondary phosphate, magnesium phosphate, magnesium sulfate, sodium chloride, ferrous sulfate, manganese sulphate, copper sulfate, calcium carbonate and the like are used.

Culture is normally performed by shake culture, culture with aeration and agitation or the like under aerobic conditions at 30°C for 6 to 24 hours. During culture, pH is maintained between 4.0 and 6.0. pH is adjusted using inorganic or organic acid, alkali solution or the like. During culture, if necessary, antibiotics such as ampicillin or tetracycline may be added to the medium.

After the completion of culture, a gene product or a substance produced by the expression product can be collected from the culture by normal protein purification techniques and the like. For example, when produced within transformed cells, the cells are disrupted by standard methods such as disruption by ultrasonication, trituration or disruption by press, so as to extract a gene product or a substance produced by the expression product. If necessary, a protease inhibitor is added. Furthermore, when the product or the substance is produced in the culture supernatant, the culture solution itself can be used. Subsequently, the solution is subjected to filtration, centrifugation or the like to remove solid mass, and then nucleic acids are removed by protamine suspension or the like if necessary.

Next, ammonium sulfate, alcohol, acetone or the like is added for fractionation. The precipitate is collected, and then a crude protein solution is obtained. The protein solution is subjected to various chromatographies, electrophoresis or the like, thereby obtaining a purified enzyme sample. A purified gene product of interest or a substance produced by the expression product can be obtained by, for example, appropriately selecting from or combining gel filtration using Sephadex, Ultrogel, Biogel or the like, ion-exchange chromatography, electrophoresis using polyacrylamide gel and the like, and fractionation methods using such as affinity chromatography or reversed-phase chromatography. However, the above culture methods and purification methods are examples, and the methods that can be used herein are not limited thereto.

In addition, for example, the amino acid sequence of a purified gene product or a substance produced by an expression product can be confirmed by a known method of amino acid analysis, such as an automatic method for determining amino acid sequences according to the Edman degradation method.

### BRIEF DESCRIPTION OF THE DRAWINGS

FIGS. 1A to 1C show the construction of a chromosome-integrating type vector, pBTRP-PDC1-LDH.
FIGS. 2A to 2B show the construction of a chromosome-integrating type vector, pBTRP-PDC1-LDH.
FIG. 3 shows the genome structure of a strain that is obtained when the yeast *Saccharomyces cerevisiae* is transformed with vector pBTRP-PDC1-LDH.
FIG. 4 shows the construction of chromosome-integrating type vectors, pAUR-LacZ-T123PDC1 (A), pAUR-LacZ-OC2PDC1 (B) and pAUR-LacZ-YPHPDC1(C).
FIGS. 5A and 5B show comparison of the gene sequences of a PDC1 promoter (983 bp) isolated from a pBTRP-PDC1-LDH-introduced strain, a PDC1 promoter (968 bp) isolated from IFO2260 strain, and a PDC1 promoter (968 bp) isolated from YPH strain.
FIG.6 shows β-galactosidase activity before subculture in transformants wherein the PDC1 promoter (983 bp) isolated from a pBTRP-PDC1-LDH-introduced strain, the PDC1 promoter (968 bp) isolated from a IFO2260 strain, and the PDC1 promoter (968 bp) isolated from a YPH strain have been inserted.
FIG.7 shows β-galactosidase activity after subculture in transformants wherein the PDC1 promoter (983 bp) isolated from a pBTRP-PDC1-LDH-introduced strain, the PDC1 promoter (968 bp) isolated from a IFO2260 strain, and the PDC1 promoter (968 bp) isolated from a YPH strain have been inserted.

### BEST MODE FOR CARRYING OUT THE INVENTION

The present invention will be further specifically described below by examples. However, the scope of the present invention is not limited by these examples.

### EXAMPLES

### [Example 1] Isolation of PDC1P fragment for the construction of pBTRP-PDC1-LDH

In this example, the promoter region (PDC1P) of the pyruvate decarboxylase 1 gene was determined and isolated. The PDC1P fragment was isolated by the PCR amplification method using the genomic DNA of the *Saccharomyces cerevisiae* YPH strain (Stratagene) as a template.

The genomic DNA of the *Saccharomyces cerevisiae* YPH strain was prepared using a Fast DNA Kit (Bio 101), which was a genome preparation kit, according to the attached protocol. The DNA concentration was measured using an Ultro spec 3000 spectral photometer (Amersham Pharmacia Biotech).

In a PCR reaction, Pyrobest DNA polymerase (TAKARA BIO) thought to have high accuracy to produce amplification fragments was used as an enzyme for amplification. The genomic DNA (50 ng/sample) of the *Saccharomyces cerevisiae* YPH strain that had been prepared by the above technique, primer DNA (50 pmol/sample) and Pyrobest DNA polymerase (0.2 units/sample) were prepared to result in a reaction of 50 µl in total. The reaction solution was subjected to a PCR amplification system (Gene Amp PCR system 9700, PE Applied Biosystems) to perform DNA amplification. Reaction conditions for the PCR amplification system consisted of 96°C for 2 minutes followed by 25 cycles of 96°C for 30 seconds, 55°C for 30 seconds and 72°C for 90 seconds, and then 4°C. The amplification fragment of the PDC1 primer was subjected to 1% TBE agarose gel electrophoresis so as to confirm the gene amplification fragment. In addition, the primer DNAs used for this reaction were synthetic DNAs (Sawady Technology), and the DNA sequences of the primers are as follows.

·Restriction enzyme BamHI site was added to the end of PDC1P-LDH-U (31 mer and Tm of 58.3°C)
: ATA TAT GGA TCC GCG TTT ATT TAC CTA TCT C (SEQ ID NO: 2)

·Restriction enzyme EcoRI site was added to the end of PDC1P-LDH-D (31 mer and Tm or 54.4 °C)
: ATA TAT GAA TTC TTT GAT TGA TTT GAC TGT G (SEQ ID NO: 3)

### [Example 2] Construction of recombinant vector containing promoter and target gene

In this example, a recombinant vector was constructed using the lactate dehydrogenase gene (LDH gene) isolated from *Bifidobacterium longum* as a target gene under the control of the pyruvate decarboxylase I gene (PDC1) promoter sequence isolated from *Saccharomyces cerevisiae.*

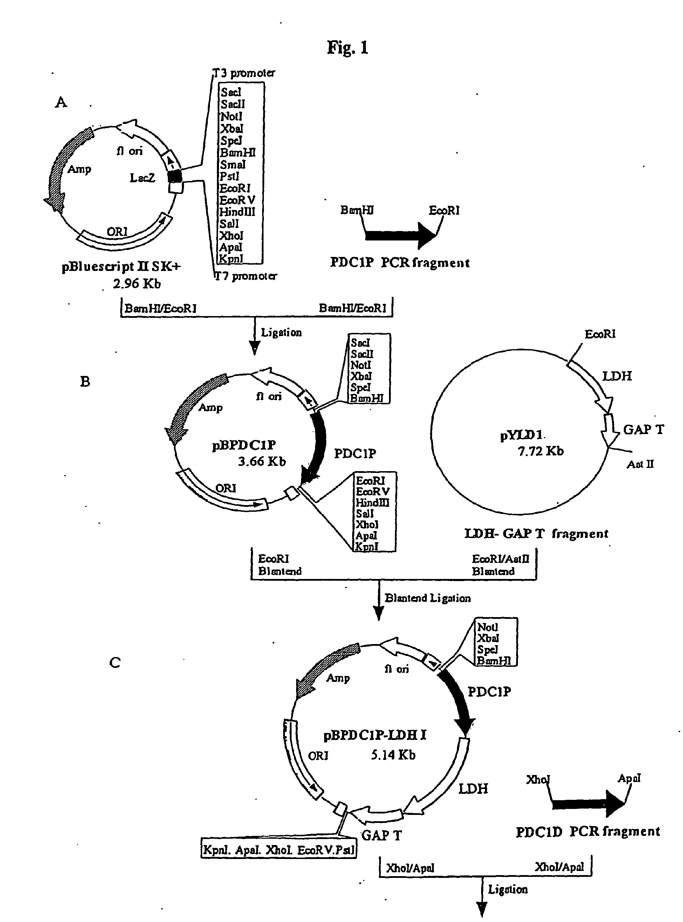

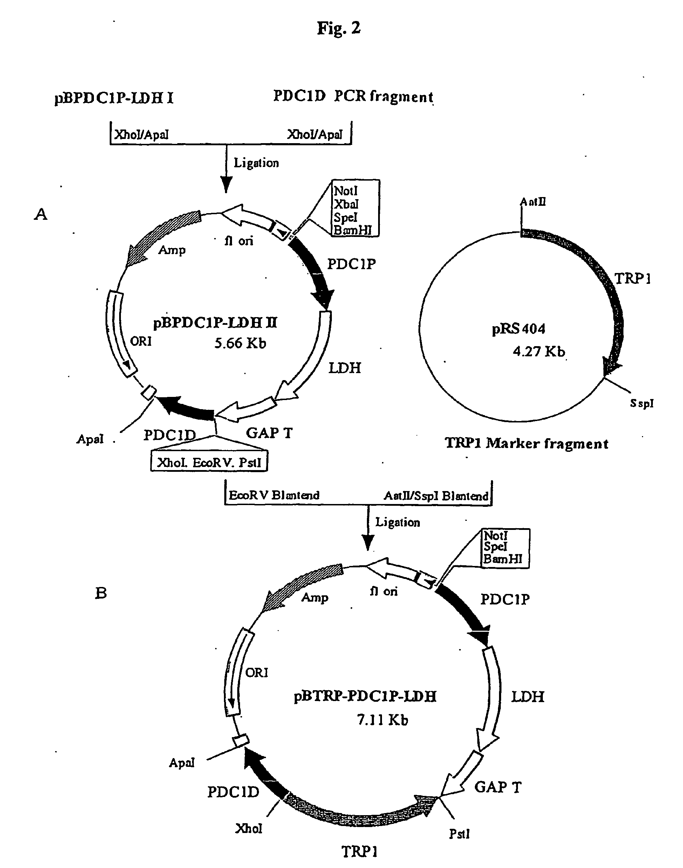

A chromosome-integrating type vector, which had been newly constructed for this example, was designated pBTRP-PDC1-LDH. An example of the construction of this vector will be described in detail below. In addition, an outline of this example is shown in Figs. 1 and 2. However, the procedures for vector construction are not limited thereto.

Upon the construction of the vector, required gene fragments (a 971 bp promoter fragment (PDC1P) of the PDC1 gene and a 518 bp fragment (PDC1D) of the downstream region of the PDC1 gene) were isolated by the PCR amplification method using the genomic DNA of *Saccharomyces cerevisiae* YPH strain as a template, as described above. Procedures for PCR amplification were as described above. For amplification of the fragment of the downstream region of the PDC1 gene, the following primers were used.

·Restriction enzyme XhoI site was added to the end of PDC1D-LDH-U (34 mer and Tm of 55.3°C)
: ATA TAT CTC GAG GCC AGC TAA CTT CTT GGT CGA C (SEQ ID NO: 4)

·Restriction enzyme *ApaI* site was added to the end of PDC1D-LDH-D (31 mer and Tm of 54.4°C)
: ATA TAT GAA TTC TTT GAT TGA TTT GAC TGT G (SEQ ID NO: 5)

Each of the gene amplification fragments of PDC1P and PDC1D obtained in the above reaction was respectively purified by ethanol precipitation treatment. Then, the PDC1P amplification fragment and the PDC1D amplification fragment were treated by restriction enzyme reaction using restriction enzymes BamHI/EcoRI and restriction enzymes *Xho*I/*Apa*I, respectively. In addition, the enzymes used below were all produced by TAKARA BIO. Furthermore, detailed manuals for a series of procedures including ethanol precipitation treatment and treatment with restriction enzymes were used according to Molecular Cloning: A Laboratory Manual second edition (Maniatis et al., Cold Spring Harbor Laboratory press. 1989).

A series of reaction procedures upon the construction of the vector was performed according to a general DNA subcloning method. Specifically, to the pBluescriptII SK+ vector (TOYOBO) that had been treated with restriction enzymes *Bam*HI/*Eco*RI (TAKARA BIO) and an alkaline phosphatase (BAP, TAKARA BIO), which was a dephosphorylase, the PDC1P fragment that had been amplified by the above PCR method and then treated with restriction enzymes was ligated by a T4 DNA Ligase reaction (Fig. 1A). The T4 DNA Ligase reaction was performed using the LigaFast Rapid DNA Ligation System (Promega) according to the attached protocols.

Next, the solution that had been subjected to the ligation reaction was then used for the transformation of competent cells. The competent cells used herein were *Escherichia coli* JM109 strain (TOYOBO), and the transformation was performed according to the attached protocols. The obtained culture solution was inoculated on an LB plate containing 100 µg/ml antibiotics (ampicillin), followed by overnight culture. The colonies that had grown were confirmed by the colony PCR method using a primer DNA of the insert fragment, and a plasmid DNA solution prepared by Miniprep was confirmed by treatment with restriction enzymes, thereby isolating the pBPDC1P vector, which was the target vector (Fig. 1B).

Subsequently, the LDH gene fragment, which had been obtained by treating the pYLD1 vector constructed by TOYOTA JIDOSHA KABUSHIKI KAISHA with restriction enzymes *Eco*RI/*Aat*II and T4 DNA polymerase, the terminus-modifying enzyme, was subcloned by procedures similar to those described above into the pBPDC1P vector, which had been similarly treated with restriction enzyme *Eco*RI and T4 DNA polymerase, the terminus-modifying enzyme, thereby preparing the pBPDC1P-LDH I vector (Fig. 1C). In addition, the above pYLD1 vector was introduced into *Escherichia coli* (name: "*E. coli* pYLD 1") and internationally deposited under the Budapest Treaty and under the accession number of FERM BP-7423 with the International Patent Organism Depositary at the National Institute of Advanced Industrial Science and Technology (1-1-1, Higashi, Tsukuba, Ibaraki, Japan); (original deposition date: October 26, 1999). Next, the vector was treated with *Xho*I/*Apa*I*,* and then an amplified PDC1D fragment was ligated thereto, thereby preparing the pBPDC1P-LDH II vector (Fig. 2A). Finally, the TRP1 marker fragment, which had been obtained by treating the pRS404 vector (Stratagene) with *Aat*II/*Ssp*I and T4 DNA polymerase, was ligated to the pBPDC1P-LDH II vector, which had been treated with *Eco*RV*,* thereby constructing a final construct, the pBTRP-PDC1-LDH vector of a type to be introduced into a chromosome (Fig. 2B).

To confirm the thus constructed chromosome-integrating type pBTRP-PDC1-LDH vector, the nucleotide sequence was determined. An ABI PRISM 310 Genetic Analyzer (PE Applied Biosystems) was used as a nucleotide sequence analyzer, and determination was performed according to the manuals attached to this analyzer so as to discover details concerning a method of preparing samples, a method of using instruments and the like. A vector DNA to be used as a sample was prepared by an alkali extraction method. The DNA was column-purified using a GFX DNA Purification kit (Amersham Pharmacia Biotech), DNA concentration was measured with an Ultro spec 3000 spectrophotometer (Amersham Pharmacia Biotech), and was then used.

### [Example 3] Introduction of recombinant vector to host

A tryptophan dependent strain of yeast, the IFO2260 strain (the strain registered at the Institute for Fermentation, Osaka), which was a host, was cultured in 10 ml of YPD medium at 30°C to a logarithmic growth phase. After harvest and washing with TE buffer, 0.5 ml of TE buffer and 0.5 ml of 0.2 M lithium acetate were added, and then shake culture was performed at 30°C for 1 hour. Subsequently, pBTRP-PDC1-LDH, which had been treated with restriction enzymes *Apa*I and *Spe*I*,* was added.

The suspension of the plasmid was shake-cultured at 30°C for 30 minutes, 150 ml of 70% polyethylene glycol 4000 was added, and then the solution was agitated well. After 1 hour of shake culture at 30°C, heat shock was given at 42°C for 5 minutes. The cells were washed and then suspended in 200 ml of water. The suspension was spread on a selection medium.

After the resulting colonies were isolated with the selection medium to obtain colonies, strains wherein LDH had been inserted downstream of the PDC1 promoter were obtained by PCR. Furthermore, spore formation was performed in media for spore formation, diploid formation was performed using the homothallic property, and then a strain wherein the above vector had been introduced into both chromosomes of the diploid was obtained.

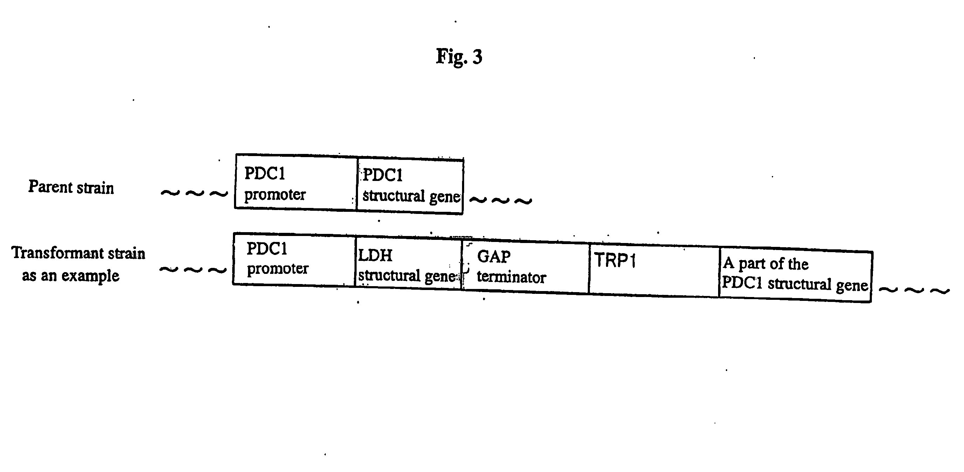

The fact that the yeast *Saccharomyces cerevisiae* had been transformed with pBTRP-PDC1-LDH shown in Fig. 2 and the gene had been inserted into the genome was confirmed by PCR. The structure of the above vector on the genome is shown in Fig. 3.

### [Example 4] Production of substance by expression product

The obtained transformant was inoculated at a cell concentration of 1 % in YPD liquid medium (glucose 10%), and then static culture was performed at 30°C for 2 days. Comparison were conducted for the amounts of lactic acid produced by (1) a strain to which no vector had been introduced, (2) a strain to which LDH had been inserted with the YEP vector (a system to which LDH had been inserted under the control of a conventional GAP promoter), and (3) a strain into which LDH had been inserted with pBTRP-PDC1-LDH (a system into which LDH had been inserted under the control of the PDC1 promoter). The results are shown in Table 1.

**Table 1 Comparison of the amounts of lactic acid produced as a result of different methods of introducing LDH and subculture**

| Method of introducing LDH | Before subculture | After subculture |
|---|---|---|
| (1) Parent strain (LDH was absent) | 0% | - |
| (2) Introduction of LDH with YEP vector (GAP promoter) | 0.4% | 0% |
| (3) Introduction of LDH with pBTRP-PDC1-LDH (PDC1 promoter) | 1.0% | 1.0% |

While the strain (1) to which no vector had been introduced produced no lactic acid, the strains (2) and (3) into which LDH had been inserted produced lactic acid. Furthermore, the strain (3) to which LDH had been inserted with the chromosome-integrating type vector under the control of the PDC1 promoter produced lactic acid at a level 2.5 times greater than that produced by the strain

### (2) into which LDH had been inserted with the YEP vector.

Moreover, for the purpose of confirming the stability of the trait introduced by the method, subculture was performed 3 times on YPD plates, and then gene transfer and the amount of lactic acid produced were examined by PCR. While the system (2) into which LDH had been inserted with the YEP vector stopped to produce lactic acid, the strain (3) which had been caused to express LDH maintained the production of lactic acid in the same amount as that produced before subculture.

Moreover, it was confirmed by PCR that there was no change in the structure on the genome. Accordingly, the system (3) wherein LDH is expressed by pBTRP-PDC1-LDH can be said to be present stably and enable the high expression of the gene.

Based on the above results, it was shown that LDH is stably and highly expressed in the case of using the chromosome-integrating type, wherein LDH had been operably linked LDH to the PDC1 promoter of the present invention.

### [Example 5] Isolation of PDC1 promoter sequence containing variant sequence

In this example and the following examples, 3 types of PDC1 promoter sequences having sequences differing in several nucleotides were isolated. Then, chromosome-integrating type vectors designed to ligate a LacZ gene following the promoter were prepared. Transformed yeast was constructed by inserting the promoter and 1 copy of the gene into the same position in the chromosome using these vectors. β-galactosidase activity of each transformed yeast was measured, and 3 types of promoter activities were compared.

In this example, the PDC1 promoter sequence was isolated by the PCR amplification method using as a template the genomic DNAs of the *Saccharomyces cerevisiae* pBTRP-PDC1-LDH-introduced strain (the strain prepared in Example 3), the IFO2260 strain (the strain registered at the Institute for Fermentaiton, Osaka) and the YPH strain (Stratagene).

The preparation method and the PCR amplification method for the genomic DNAs of each *Saccharomyces cerevisiae* strain (pBTRP-PDC1-LDH-introduced strain, IFO2260 strain and YPH strain) were performed by techniques similar to those employed in Examples 1 and 2.

In addition, the nucleotide sequences of the primer DNAs used for reaction are as follows.

Amplification of the PDC1 promoter isolated from the pBTRP-PDC1-LDH-introduced strain

Restriction enzyme *SalI* site was added to the end of PDC1 PrFrag-U2 (32 mer and Tm of 64.4°C)
: AAA TTT GTC GAC AAG GGT AGC CTC CCC ATA AC (SEQ ID NO: 6)

Restriction enzyme *Sal*I site was added to the end of PDC1 PrFrag-D2 (31 mer and Tm of 61.1°C)
: ATA TAT GTC GAC GAG AAT TGG GGG ATC TTT G (SEQ ID NO: 7)

Amplification of IFO2260 strain-derived and YPH strain-derived PDC1 promoters

Restriction enzyme SalI site was added to the end of PDC1 PrFrag-U2 (32 mer and Tm of 64.4°C)
: AAA TTT GTC GAC AAG GGT AGC CTC CCC ATA AC (SEQ ID NO: 6)

.Restriction enzyme *Sal*I site was added to the end of PDC1 PrFrag-D (43 mer and Tm of 62.5°C)
: TTT AAA GTC GAC TTT GAT TGA TTT GAC TGT GTT ATT TTG CGT G (SEQ ID NO: 8)

### [Example 6] Construction of vector containing variant promoter sequence for analyzing β-galactosidase

In this example, under the control of the 3 types of isolated PDC1 promoter sequences, vectors were constructed wherein reporter genes had been ligated. As a reporter gene, β-galactosidase gene (LacZ gene) was used.

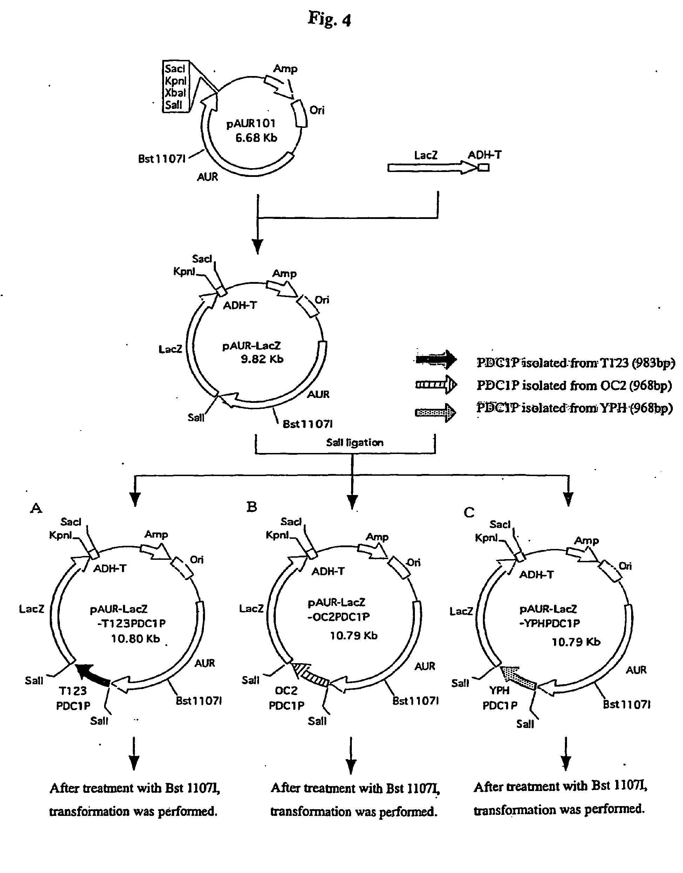

Vectors of a type to be introduced into a chromosome that had been newly constructed for this example were designated pAUR-LacZ-T123PDC1, pAUR-LacZ-OC2PDC1 and pAUR-LacZ-YPHPDC1. An example of the construction of a vector will be described in detail below. In addition, an outline of this example is shown in Fig. 4. However, the procedures for the construction of the vectors are not limited to this outline.

A series of reaction procedures for the construction of the vectors was performed according to a general DNA subcloning method. pSV-β-Galactosidase Control Vector (Promega) was excised with restriction enzymes so as to obtain a LacZ fragment. Then, the fragment was blunt-ended, so that the pAUR-LacZ vector was prepared. The thus constructed pAUR-LacZ vector was treated with *Sal*I (TAKARA BIO) and an Alkaline Phosphatase (BAP, TAKARA BIO), which was a dephosphorylase. Next, 3 types of promoter sequences obtained in Example 5, that is, the PDC1 promoter (983 bp) isolated from the pBTRP-PDC1-LDH-introduced strain, the PDC1 promoter (968 bp) isolated from the IFO2260 strain, and the PDC1 promoter (968 bp) isolated from the YPH strain, were each treated with restriction enzyme Sa*l*I (TAKARA BIO), and then ligated to the pAUR-LacZ vector by a T4 DNA Ligase reaction. T4 DNA Ligase reaction was performed using a LigaFast Rapid DNA Ligation System (Promega) according to the attached protocols.

Competent cells were transformed using the thus obtained Ligation reaction solution, and then target construction vectors were obtained by the colony PCR method. The above series of procedures were performed by techniques similar to those employed in Example 2.

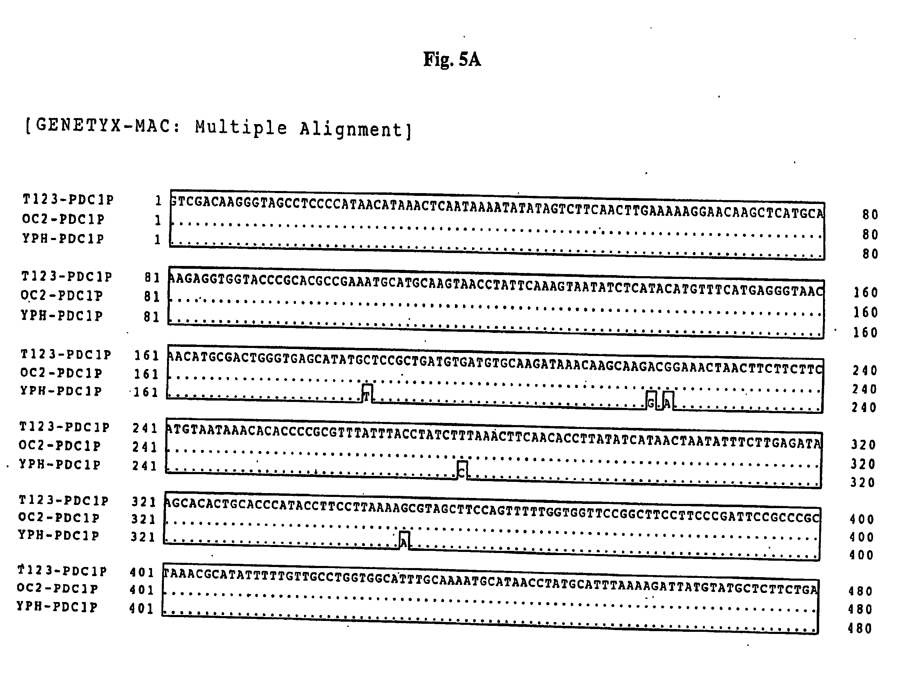

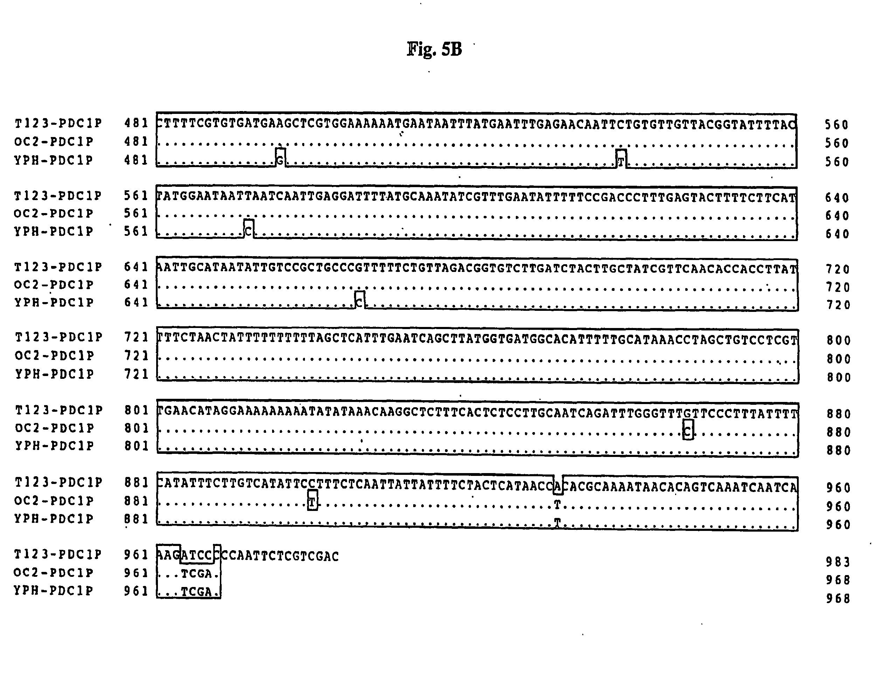

Nucleotide sequence analysis was performed for the constructed vectors, and then the gene sequences of the PDC1 promoter (983 bp) isolated from the pBTRP-PDC1-LDH-introduced strain, the PDC1 promoter (968 bp) isolated from the IFO2260 strain, and the PDC1 promoter (968 bp) isolated from the YPH strain were compared. The results of the comparison of the sequences are shown in Figs. 5A and B. In addition, nucleotide sequence analysis was performed by procedures similar to the techniques employed in Example 2.

The PDC1 promoter (983 bp) isolated from the pBTRP-PDC1-LDH-introduced strain differed from the PDC1 promoter sequence (971 bp) comprising the nucleotide sequence represented by SEQ ID NO: 1 by 12 nucleotides. Specifically, the PDC1 promoter (983 bp) isolated from the pBTRP-PDC1-LDH-introduced strain was composed of a sequence wherein restriction enzyme *Sal* I site (GTCGAC) was added to both ends of the promoter sequence of SEQ ID NO: 1.

Furthermore, the IFO2260 strain-derived PDC1 promoter (968 bp) differed from the PDC1 promoter sequence comprising the nucleotide sequence represented by SEQ ID NO: 1 by 30 nucleotides, wherein specifically the guanine (G) at position 861 of the promoter sequence of SEQ ID NO: 1 was substituted with cytosine (C), the cytosine (C) at position 894 was substituted with thymine (T), the adenine at position 925 was substituted with thymine (T), and a sequence (GATCCCCCAATTCTC) of 15 nucleotides was added following the nucleotide at position 972. Furthermore, the IFO2260 strain-derived PDC1 promoter sequence was composed of a sequence wherein restriction enzyme *Sal*I site (GTCGAC) was added to both ends of the promoter sequence of SEQ ID NO: 1.

The YPH strain-derived PDC1 promoter (968 bp) differed from the PDC1 promoter sequence comprising the nucleotide sequence represented by SEQ ID NO: 1 by 37 nucleotides, wherein, specifically, the cytosine (C) at position 179 of the promoter sequence of SEQ ID NO: 1 was substituted with thymine (T), the adenine (A) at position 214 was substituted with guanine (G), the guanine (G) at position 216 was substituted with adenine (A), the thymine (T) at position 271 was substituted with cytosine (C), the guanine (G) at position 344 was substituted with adenine (A), the adenine (A) at position 490 was substituted with guanine (G), the cytosine (C) at position 533 was substituted with thymine (T), the thymine (T) at position 566 was substituted with cytosine (C), the guanine (G) at position 660 was substituted with cytosine (C), the adenine (A) at position 925 was substituted with thymine (T), and a sequence (GATCCCCCAATTCTC) of 15 nucleotides was added following the nucleotide at position 972. Furthermore, the YPH strain-derived PDC1 promoter sequence was composed of a sequence wherein restriction enzyme Sa*l*I site (GTCGAC) was added to both ends of the promoter sequence of SEQ ID NO: 1.

### [Example 7] Introduction of recombinant vector into host

A tryptophan dependent strain of yeast, the IFO2260 strain (the strain registered at the Institute for Fermentation, Osaka), as a host was cultured in 10 ml of YPD medium at 30°C to a logarithmic growth phase. After harvest and washing with TE buffer, 0.5 ml of TE buffer and 0.5 ml of 0.2 M lithium acetate were added, and then shake culture was performed at 30°C for 1 hour. Subsequently, pAUR-LacZ-T123PDC1P, pAUR-LacZ-YPHPDC1P and pAUR-LacZ-OC2PDC1P, which had been treated with restriction enzyme *Bst*1107 I (TAKARA BIO), were added.

The suspension of the plasmid was shake-cultured at 30°C for 30 minutes, 150 µl of 70% polyethylene glycol 4000 was added, and then the solution was agitated well. The solution was subjected to 1 hour of shake culture at 30°C, and then heat shock was given at 42°C for 5 minutes. The cells were cultured in 1 ml of YPD medium at 30°C for 12 hours. The culture solution was washed, and then suspended in 200 µl of sterilized water. The suspension was then spread onto aureobasidin A selection medium. The concentration of aureobasidin A added to the medium was 0.4 µg/ml.

The obtained colonies were isolated using the aureobasidin A selection medium, and then the PCR method was performed for the resultant colonies, thereby obtaining a target strain.

### [Example 8] Measurement of β-galactosidase activity in gene recombinant strain

β-galactosidase activity was measured for the above transformant and non-transformant. Each strain was cultured in 2 ml of YPD liquid medium (glucose 2%) at 30°C for 20 hours. They were harvested and 500 µl of 50 mM Tris-HCl and glass beads (425 to 600 microns Acid Washed, SIGMA) were added, and vortexed for 15 minutes at 4°C.

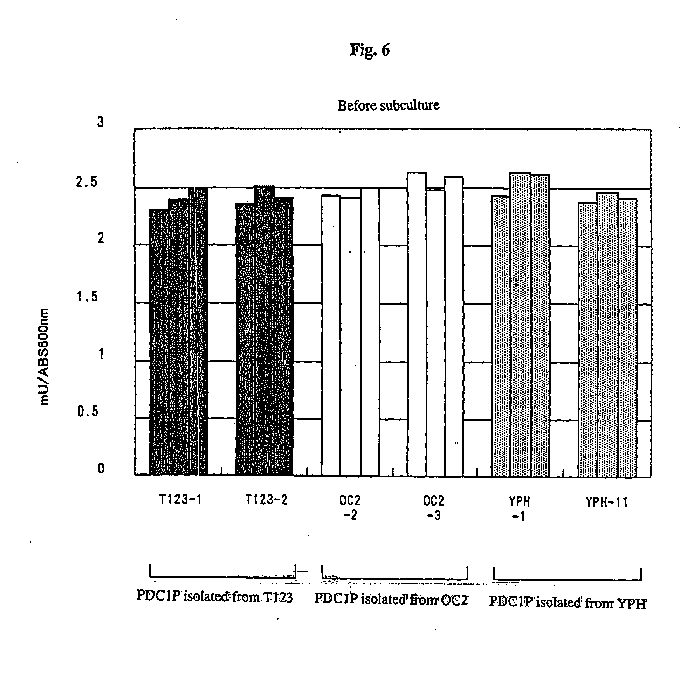

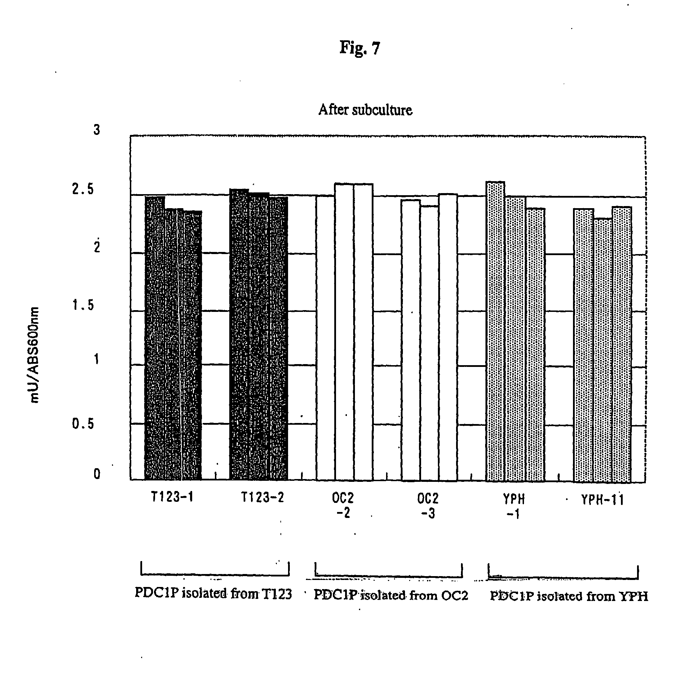

The supernatant of this solution was collected by centrifugation, and then β-galactosidase activities in these supernatants were measured. Activity measurement was performed using β-Galactosidase Enzyme Assay System (Promega) according to the attached protocols. The value of activity (ABS 600 nm=1.0) was calculated, and the results are shown in Fig. 6 (before subculture) and Fig. 7 (after subculture).

Based on the above results, it was revealed that even a PDC1 promoter sequence having a sequence of several tens of nucleotides added thereto or having a different sequence possesses stable promoter activity. Therefore, it can be said that the promoter of a gene wherein the autoregulation mechanism is present, or the promoter of a gene that is not essential for growth or fermentation in a host organism can be utilized, even if it does not have a full-length sequence.

### INDUSTRIAL APPLICABILITY

According to the present invention, a lactate dehydrogenase gene can be stably introduced and highly expressed in Saccharomyces cerevisiae without affecting growth and fermentation of the host. Hence, an effective tools for producing a substance and altering or analyzing the function, is provided.

### SEQUENCE LISTING FREE TEXT

SEQ ID NO: 1: Synthetic DNA
SEQ ID NO: 2: Synthetic DNA
SEQ ID NO: 3: Synthetic DNA
SEQ ID NO: 4: Synthetic DNA
SEQ ID NO: 5: Synthetic DNA
SEQ ID NO: 6: Synthetic DNA
SEQ ID NO: 7: Synthetic DNA
SEQ ID NO: 8: Synthetic DNA

## Claims

1. A method for producing lactic acid by expressing a lactate dehydrogenase gene in *Saccharomyces cerevisiae* yeast without affecting growth and fermentation of the yeast, which comprises disrupting the pyruvate decarboxylase 1 gene on a genome of the *Saccharomyces cerevisiae* yeast; culturing in a culture medium the yeast, wherein the lactate dehydrogenase gene is inserted into the yeast genome using a chromosome-integrating type vector containing the promoter of the pyruvate decarboxylase 1 gene from *Saccharomyces cerevisiae,* and expressing the lactate dehydrogenase gene.

2. The production method of claim 1, wherein the chromosome-integrating type vector is pBTRP-PDC1-LDH having the following structure:

## Patentansprüche

1. Verfahren für die Herstellung von Milchsäure durch Expression eines Lactatdehydrogenase-Gens in der Hefe *Saccharomyces cerevisiae* ohne Beeinträchtigung von Wachstum und Fermentation der Hefe, welches das Unterbrechen des Gens der Pyruvatdecarboxylase 1 in einem Genom der Hefe *Saccharomyces cerevisiae;* das Kultivieren der Hefe in einem Kulturmedium, wobei das Lactatdehydrogenase-Gen in das Hefegenom unter Verwendung eines Vektors vom Chromoson-integrierenden Typ, der den Promoter des Gens der Pyruvatdecarboxylase 1 von *Saccharomyces cerevisiae* enthält, eingefügt wird, und das Exprimieren des Lactatdehydrogenase-Gens umfasst.

2. Herstellungsverfahren nach Anspruch 1, wobei der Vektor vom Chromoson-integrierenden Typ pBTRP-PDCC1-LDH mit der folgenden Struktur ist:

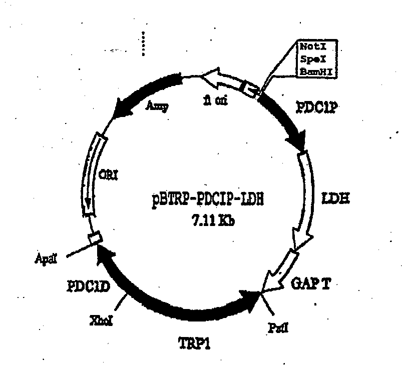

## Revendications

1. Procédé pour produire de l'acide lactique par l'expression d'un gène lactate déshydrogénase dans une levure *Saccharomyces cerevisiae* sans affecter la croissance et la fermentation de la levure, qui comprend l'interruption du gène pyruvate décarboxylase 1 sur un génome de la levure *Saccharomyces cerevisiae ;* la culture dans un milieu de culture de la levure, dans laquelle le gène lactate déshydrogénase est inséré dans le génome de la levure en utilisant un vecteur de type intégration de chromosome contenant le promoteur du gène pyruvate décarboxylase 1 de *Saccharomyces cerevisiae,* et l'expression du gène lactate déshydrogénase.

2. Procédé de production selon la revendication 1, dans lequel le vecteur de type intégration de chromosome est pBTRP-PDC1-LDH ayant la structure suivante :
